# FASCICULE DE BREVET EUROPEEN

(11) **EP 0 641 341 B1**
(45) Date de publication et mention de la délivrance du brevet: **01.03.2000**
(21) Numéro de dépôt: 92922701.5
(22) Date de dépôt: 08.10.1992
(51) Int. Cl.: C07D 471/04

(54) **PROCEDE DE PREPARATION DE BENZO [b]NAPHTYRIDINES**
VERFAHREN ZUR HERSTELLUNG VON BENZO[b]NAPHTHYRIDINEN
METHOD FOR PREPARING BENZO [b]NAPHTHYRIDINES

(30) Priorité: 10.10.1991 FR 9112479
(43) Date de publication de la demande: 08.03.1995
(73) Titulaire: RHONE-POULENC RORER S.A., 92160 Antony (FR)
(72) Inventeur: DAUBIE, Christophe, F-75005 Paris (FR); LEGRAND, Jean-Jacques, F-75013 Paris (FR); PEMBERTON, Clive, Romfold, Essex RM3 0XB (GB)
(86) Numéro de dépôt international: FR9200937
(87) Numéro de publication internationale: WO9307145

(56) Documents cités:
- JOURNAL OF MEDICINAL CHEMISTRY. vol. 18, no. 10, 1975, WASHINGTON US pages 1038 - 1041 A. A. SANTILLI 'Synthesis and antibacterial evaluation of 1,2,3,4-tetrah ydro-4-oxo-1,8-naphthyridine-3-carboxylic acid esters, carbonitriles and carboxamides'

## Description

La présente invention concerne un nouveau procédé de préparation de benzo [b] naphtyridines de formule générale : 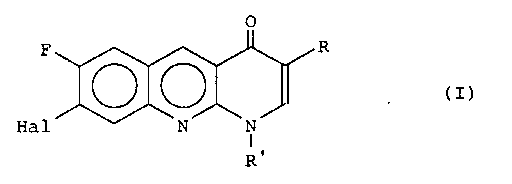 dans laquelle R est un radical carboxy, alcoyloxycarbonyle, cyano, carbamoyle, alcoylcarbamoyle, benzylcarbamoyle, hydroxyéthylcarbamoyle, dialcoylaminoéthylcarbamoyle ou dialcoylcarbamoyle dont les parties alcoyle peuvent éventuellement former avec l'atome d'azote auquel elles sont rattachées, un hétérocycle à 5 ou 6 chaînons contenant éventuellement un autre hétéroatome choisi parmi l'oxygène, le soufre ou l'azote et éventuellement substitué sur l'azote par un radical alcoyle, R' est un atome d'hydrogène ou un radical alcoyle, fluoroalcoyle, carboxyalcoyle, cycloalcoyle (contenant 3 à 6 atomes de carbone), fluorophényle, difluorophényle, alcoyloxy ou alcoylamino, et Hal est un atome d'halogène, ainsi que leurs sels lorsqu'ils existent.

Les benzo[b]naphtyridines de formule générale (I) ainsi que leur préparation et leur utilisation pour la préparation d'agents antibactériens ont été décrites dans les brevets US 4 970 213 et US 4 990 515.

Le J. Med. Chem., 18(10), 1038-41 (1975) décrit la préparation de l'acide nalidixique par action de l'amine R,NH-CH₂-CH₂-Z sur la chloro-2 pyridine correspondante. Cependant l'acide nalidixique ne comporte aucun substituant halogéné.

Il a été trouvé que les dérivés de la benzo [b] naphtyridine de formule générale (I) peuvent être préparés à partir d'un dérivé de la quinoléine de formule générale : 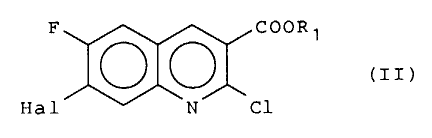 dans laquelle Hal est défini comme ci-dessus et R₁ est un atome d'hydrogène ou un radical alcoyle, en effectuant les opérations suivantes :
1) condensation en milieu basique d'une amine de formule générale :

   R' -NH-CH2-CH2-R'' (III)

   dans laquelle R' est défini comme précédemment et R'' est un radical alcoyloxycarbonyle, cyano, carbamoyle, alcoylcarbamoyle, benzylcarbamoyle, hydroxyéthylcarbamoyle, dialcoylaminoéthylcarbamoyle ou dialcoylcarbamoyle dont les parties alcoyle peuvent éventuellement former avec l'atome d'azote auquel elles sont rattachées, un hétérocycle à 5 ou 6 chaînons contenant éventuellement un autre hétéroatome choisi parmi l'oxygène, le soufre ou l'azote et éventuellement substitué sur l'azote par un radical alcoyle, sur une chloro fluoro quinoléine de formule générale (II) dans laquelle R₁ est un radical alcoyle,
2) cyclisation en milieu basique de la fluoroquinoléine, ainsi obtenue, de formule générale : 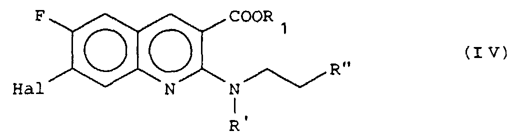 dans laquelle R', R'' et Hal sont définis comme précédemment et R₁ est défini comme ci-dessus en 1),
3) oxydation par l'eau oxygénée, éventuellement en présence d'iodure de potassium, de la tétrahydro-1,2,3,4 benzo[b]naphtyridine-1,8 de formule générale : 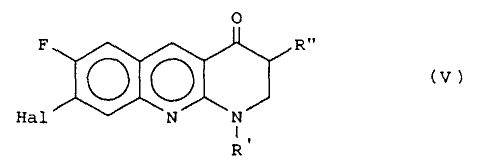 dans laquelle Hal R' et R'' sont définis comme précédemment, puis éventuellement transformation de l'ester de formule générale (I), ainsi obtenu, en un acide pour lequel R est un radical carboxy et éventuellement transformation de l'acide obtenu en un sel.

Le nouveau procédé selon la présente invention est particulièrement intéressant du fait qu'il permet d'obtenir des rendements améliorés et aussi du fait qu'il évite de passer intermédiairement par des produits instables.

Dans les formules générales citées ci-dessus ou qui seront citées ci-après, il est entendu que les radicaux alcoyle sont droits ou ramifiés et contiennent 1 à 4 atomes de carbone. Par ailleurs le symbole Hal est avantageusement choisi parmi le chlore ou le fluor.

La condensation du β-amino ester de formule générale (III) s'effectue sur le dérivé de la quinoléine de formule générale (II) dont le cas échéant la fonction acide est protégée à l'état d'ester. On opère en milieu basique dans un solvant organique tel qu'un hydrocarbure aromatique (toluène par exemple), un amide (diméthylformamide, N-méthyl pyrrolidone par exemple), un éther (tétrahydrofuranne par exemple), un sulfoxyde (diméthylsulfoxyde par exemple), un solvant chloré (dichlorométhane, dichloroéthane, chlorobenzène par exemple) ou un alcool à une température comprise entre -10 et 120°C.

A titre d'exemple les bases utilisées peuvent être choisies parmi les carbonates alcalins (carbonate de sodium ou de potassium), les alcoolates ou un hydrure alcalin (hydrure de sodium).

Il est entendu que dans l'alternative où le symbole R' représente un radical carboxyalcoyle, ce dernier est protégé préalablement à la réaction. L'élimination du radical protecteur est effectuée de préférence après l'étape 3) d'oxydation. La protection et la libération de la fonction acide s'effectuent selon les méthodes habituelles qui n'altèrent pas le reste de la molécule. Notamment selon les méthodes qui sont citées dans les références ci-après.

La réaction de cyclisation du produit de formule générale (IV) s'effectue en milieu basique. On opère avantageusement à une température comprise entre -70 et 120°C de préférence entre -30 et 120°C) en présence d'une base comme un alcoolate (éthylate de sodium, méthylate de sodium, t.butylate de potassium par exemple), un hydrure alcalin (hydrure de sodium par exemple), ou encore un hydroxyde alcalin en opérant par transfert de phase. On opère avantageusement dans un solvant aprotique polaire (par exemple diméthylformamide, tétrahydrofuranne) ou dans un alcool (éthanol, méthanol par exemple) dans un glyme ou dans un glycol (éthylène glycol par exemple). Lorsque l 'on effectue la réaction par transfert de phase, on opère avantageusement dans un solvant chloré comme le chlorure de méthylène, la base étant en solution dans la phase aqueuse.

L'oxydation s'effectue par l'eau oxygénée, éventuellement en présence d'iodure de potassium, dans un solvant organique tel qu'un alcool (éthanol par exemple), à une température comprise entre 0 et 120°C. Il est également possible d'opérer en milieu biphasique dans un mélange eau/solvant chloré (dichlorométhane, dichloroéthane...).

Le dérivé de la quinoléine de formule générale (II) peut être obtenu selon la méthode décrite dans le brevet US 4 970 213 suivie éventuellement de l'estérification du produit obtenu selon les méthodes habituelles.

Le dérivé de la quinoléine de formule générale (II) peut également être obtenu par chloration d'un dérivé de la quinoléine de formule générale : 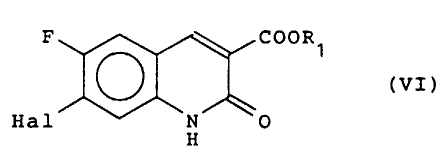 dans laquelle R₁ et Hal sont définis comme précédemment.

La chloration s'effectue au moyen des agents de chloration connus qui n'altèrent pas le reste de la molécule. Notamment on opère par action du chlorure de phosphoryle, du chlorure de sulfuryle ou du pentachlorure de phosphore à une température comprise entre 0 et 150°C. Il est entendu que lorsque l'on opère à partir d'un dérivé de la quinoléine de formule générale (VI) pour lequel R₁ est un atome d'hydrogène, il est nécessaire de protéger préalablement la fonction acide. Lorsque l'on veut obtenir l'acide de formule générale (II) dans laquelle R₁ est un atome d'hydrogène, on effectue l'hydrolyse de l'ester obtenu par toute méthode connue qui n'altère pas le reste de la molécule. La protection de la fonction acide et l'élimination du radical protecteur peuvent être réalisées par tout groupement et toute méthode compatible et dont la mise en oeuvre et l'élimination n'altèrent pas le reste de la molécule. Notamment, on opère selon les méthodes décrites par T.W. GREENE, Protective Groups in Organic Synthesis, A. Wiley Interscience Publication (1981), ou par Mc OMIE, Protective Groups in Organic Chemistry, Plenum Press (1973).

Le dérivé de la quinoléine de formule générale (VI) peut être préparé par cyclisation en milieu acide d'un dérivé nitré de formule générale : 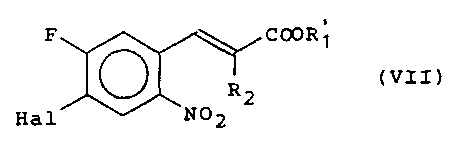 dans laquelle Hal est défini comme précédemment, R'₁ est défini comme R₁ à l'exception de représenter un atome d'hydrogène, et R₂ est défini comme R'₁ ou représente un radical carbamoyle ou cyano, suivie éventuellement de la libération de la fonction acide si l'on veut obtenir un dérivé de la quinoléine pour lequel R₁ est un atome d'hydrogène.

Le traitement en milieu acide s'effectue en présence de fer, à une température comprise entre 0 et 130°C, au moyen de tout acide organique ou minéral qui n'altère pas le reste de la molécule. A titre d'exemple on opère au moyen de l'acide acétique ou de l'acide formique, il est également possible d'opérer au moyen d'acide chlorhydrique dilué ou d'acide sulfurique dilué en milieu hydro-alcoolique. Il est bien entendu que le choix de l'acide est fonction du produit attendu. Dans le cas où l'on veut obtenir l'acide de formule générale (VI), il est avantageux d'opérer dans un acide plus fort, dans des conditions où l'hydrolyse de l'ester s'effectue simultanément; il peut également être avantageux d'opérer à partir du produit de formule générale (VII) pour lequel R₂ est cyano. Il est bien entendu que dans les cas où l'on a obtenu l'ester et où l'on veut obtenir l'acide de formule générale (VI) pour lequel R₁ est un atome d'hydrogène, l'hydrolyse de l'ester peut être également mise en oeuvre après la réaction de cyclisation, par toute méthode connue pour obtenir un acide à partir d'un ester sans toucher au reste de la molécule.

Le cas échéant l'hydrolyse de l'ester s'effectue en milieu acide, par exemple en présence d'acide chlorhydrique, d'acide sulfurique ou méthanesulfonique. Elle peut aussi être effectuée en milieu hydroalcoolique basique (soude, potasse par exemple).

Le dérivé nitré de formule générale (VII) peut être préparé par action d'un dérivé de l'acide malonique de formule générale :

R₂-CH-COOR'₁ (VIII)

dans laquelle R'₁ et R₂ sont définis comme précédemment, sur un dérivé du nitrobenzaldéhyde de formule générale : 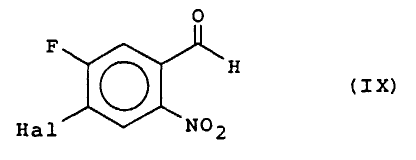 dans laquelle Hal est défini comme précédemment.

La réaction s'effectue généralement en milieu basique [par exemple en présence d'un bicarbonate alcalin (bicarbonate de sodium), d'un hydrure (hydrure de sodium) ou d'un alcoolate à une température comprise entre 0 et 150°C, dans un solvant organique tel qu'un anhydride (anhydride acétique par exemple) ou tel qu'un amide (diméthyl-formamide, N-méthylpyrrolidone par exemple) en opérant en présence de tamis moléculaires ou de tout autre agent desséchant ou encore dans un mélange de solvants comme un mélange solvant aprotique polaire/anhydride acétique (diméthylformamide/anhydride acétique, N-méthyl pyrrolidone/anhydride acétique par exemple). Il est également possible d'opérer en milieu biphasique. Il n'est pas indispensable d'isoler le produit de formule générale (VII) pour le mettre en oeuvre dans la réaction suivante.

Le fluoronitrobenzaldéhyde de formule générale (IX) est obtenu par nitration du fluorobenzaldéhyde de formule générale : 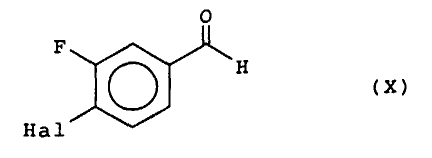 dans laquelle Hal est défini comme précédemment.

La réaction s'effectue avantageusement par l'acide nitrique concentré sous forme d'un mélange sulfonitrique, ou d'un mélange acide nitrique/acide acétique, à une température comprise entre 0 et 90°C.

Le chloro-4 fluoro-3 benzaldéhyde peut être préparé selon la méthode décrite dans la demande européenne EP 269 942.

Selon l 'invention, les dérivés de benzonaphtyridine de formule générale (I) dans laquelle R est un radical cyano, carbamoyle, alcoylcarbamoyle, benzylcarbamoyle, hydroxyéthylcarbamoyle, dialcoylaminoéthylcarbamoyle ou dialcoylcarbamoyle dont les parties alcoyle peuvent éventuellement former avec l'atome d'azote auquel elles sont rattachées, un hétérocycle à 5 ou 6 chaînons contenant éventuellement un autre hétéroatome choisi parmi l'oxygène, le soufre ou l'azote et éventuellement substitué sur l'azote par un radical alcoyle, sont des produits nouveaux.

Les dérivés de la benzo[b]naphtyridine de formule générale (I) obtenus selon le procédé de la présente invention sont spécialement intéressants du fait qu'ils conduisent à une famille d'agents antibactériens particulièrement actifs, définis par la formule générale : 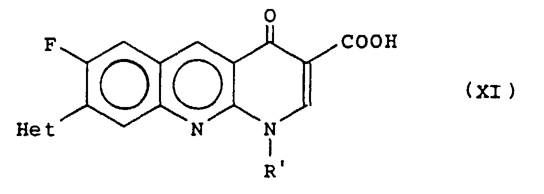 dans laquelle soit R' (qui représente un radical alcoyle, fluoroalcoyle, cycloalcoyle contenant 3 à 6 atomes de carbone, alcoyloxy ou alcoylamino) et Het (qui est un radical hétérocyclyle azoté), sont tels que définis pour les substituants en position -1 et -8, dans la demande européenne EP 431 991 et le brevet US 5 004 745, soit R' est défini comme précédemment pour la formule générale (I) et Het est un radical azétidinyle-1 substitué [en position -3 par un radical R₂ qui peut être un atome d'hydrogène ou un radical hydroxy, amino, alcoylamino dont la partie alcoyle est éventuellement substituée par un radical amino ou hydroxy ou peut représenter un radical dialcoylamino dont les parties alcoyle peuvent éventuellement former avec l'atome d'azote auquel elles sont rattachées, un hétérocycle à 5 ou 6 chaînons contenant éventuellement un autre hétéroatome choisi parmi l'azote, l'oxygène ou le soufre, ou peut représenter un radical cycloalcoylamino contenant 3 à 6 chaînons, ou un radical alcanoylamino, N-alcoyl N-alcanoyl amino ou aminoalcoyl phénylamino, et substitué en positions -2 et -3 par des radicaux R₃ et R₄ identiques ou différents qui représentent des atomes d'hydrogène, des radicaux alcoyle, alcènyle contenant 2 à 4 atomes de carbone, phényle, phényle substitué par un atome d'halogène, ou par un radical alcoyle, alcoyloxy, hydroxy, nitro, amino, alcoylamino, dialcoylamino ou halogénoalcoyle, ou bien substitué en position -2 par des radicaux R₃ et R₄ qui représentent des radicaux alcoyle], les radicaux alcoyle et alcanoyle étant droits ou ramifiés et contennant 1 à 4 atomes de carbone.

Les dérivés de la benzo[b]naphtyridine-1,8 de formule générale (XI) sont obtenus par condensation de l'hétérocycle Het sur la benzo[b]naphtyridine de formule générale (I) en opérant selon ou par analogie avec les méthodes décrites dans la demande européenne EP 431 991 et le brevet US 5 004 745 puis le cas échéant transformation de l'ester, de l'amide ou du nitrile obtenu en un acide de formule générale (XI). Les activités des dérivés de la benzonaphtyridine de formule générale (XI) ont été décrites dans la demande européenne et le brevet américain cités ci-dessus. Les dérivés de la benzonaphtyridine de formule générale (XI) pour lesquels Het est un radical azétidinyle présentent également des propriétés antibactériennes. Ils manifestent une activité remarquable in vitro et in vivo sur les germes gram positifs et aussi sur les germes gram négatifs. In vitro, ils sont actifs à une concentration comprise entre 0,06 et 4 µg/cm³ sur staphylococcus aureus IP 8203 et à une concentration comprise entre 0,25 et 20 µg/cm³ sur Escherichia coli souche NIHJ JC2. In vivo, ils sont actifs sur les infections expérimentales de la souris à staphylococcus aureus IP 8203 à des doses comprises entre 10 et 200 mg/kg par voie orale.

Les dérivés de la benzo[b]naphtyridine de formule générale (I) obtenus selon le procédé de la présente invention peuvent être éventuellement purifiés par des méthodes physiques telles que la cristallisation ou la chromatographie.

Lorsque le radical R est un radical carboxy, les dérivés de la benzo[b]naphtyridine de formule générale (I) peuvent être transformés en sels métalliques ou en sels d'addition avec les bases azotées selon les méthodes connues en soi. Ces sels peuvent être obtenus par action d'une base métallique (par exemple alcaline ou alcalino-terreuse), de l'ammoniac ou d'une amine sur un produit selon l'invention dans un solvant approprié tel qu'un alcool, un éther ou l'eau ou par réaction d'échange avec un sel d'un acide organique. Le sel formé précipite après concentration éventuelle de sa solution, il est séparé par filtration, décantation ou lyophilisation.

Comme exemples de sels, peuvent être cités les sels avec les métaux alcalins (sodium, potassium, lithium) ou avec les métaux alcalinoterreux (magnésium, calcium), le sel d' ammonium, les sels de bases azotées (éthanolamine, diéthanolamine, triméthylamine, triéthylamine, méthylamine, propylamine, diisopropylamine, NN-diméthyléthanolamine, benzylamine, dicyclohexylamine, N-benzyl phénéthylamine, NN'-dibenzyléthylènediamine, diphénylènediamine, benzhydrylamine, quinine, choline, arginine, lysine, leucine, dibenzylamine), ainsi que les sels d'addition avec des acides minéraux (chlorhydrates, bromhydrates, sulfates, nitrates, phosphates) ou organiques (succinates, fumarates, maléates, méthanesulfonates, p.toluènesulfonates, iséthionates).

Les exemples suivants donnés à titre non limitatif illustrent la présente invention.

### Exemple 1

### Préparation de l'éthoxycarbonyl-3 difluoro-6,7 (N-méthyl N-β éthoxycarbonyléthyl) amino-2 quinoléïne :

A une solution de 72 g de chloro-2 éthoxycarbonyl-3 difluoro-6,7 quinoléïne et 45,1 g de N-méthyl, N-β éthoxycarbonyléthyl amine dans 750 cm³ de toluène, on ajoute 56,2 g de carbonate de sodium. La suspension obtenue est chauffée à environ 90°C puis agitée 4 heures à cette température. Le mélange réactionnel est ensuite refroidi à environ 20°C puis lavé par 3 fois 400 cm³ d'eau. La phase organique est concentrée à sec sous pression réduite (20 kPa) à environ 50°C. On obtient 94 g d'éthoxycarbonyl-3 difluoro-6,7 (N-méthyl N-β éthoxycarbonyléthyl amino)-2 quinoléïne sous forme d'une huile utilisée sans autre purification pour les étapes ultérieures.

### Préparation de l'éthoxycarbonyl-3 difluoro-7,8 méthyl-1 oxo-4 tétrahydro-1,2,3,4 benzo[b]naphtyridine-1,8 :

A une solution de 26,6 g d'éthylate de sodium portée à reflux dans 900 cm³ d'éthanol absolu on ajoute en 80 minutes, une solution de 94 g d'éthoxycarbonyl-3 difluoro-6,7 (N-méthyl N-β éthoxycar-bonyléthyl) amino-2 quinoléïne dans 300 cm³ d'éthanol absolu. La suspension obtenue, toujours à reflux, est agitée 15 minutes supplémentaires. On verse ensuite, en 30 minutes, 38 cm³ d'acide acétique glacial. Le mélange réactionnel est agité 15 minutes supplémentaires puis on verse en 45 minutes, toujours à reflux, 500 cm³ d'eau. La suspension obtenue est refroidie à 20°C environ. Le précipité est essoré à 20°C environ et lavé par 2 fois 300 cm³ d'eau. Le produit humide est séché sous pression réduite (20 kPa) à environ 60°C. On isole 71,5 g d'éthoxycarbonyl-3 difluoro-7,8 méthyl-1 oxo-4 tétrahydro-1,2,3,4 benzo[b]naphtyridine-1,8 sous forme d'un solide jaune fondant à 188°C.

### Préparation de l'éthoxycarbonyl-3 difluoro-7,8 méthyl-1 oxo-4 dihydro-1,4 benzo[b]naphtyridine-1,8 :

A une suspension de 71 g d'éthoxycarbonyl-3 difluoro-7,8 méthyl-1 oxo-4 tétrahydro-1,2,3,4 benzo[b]naphtyridine-1,8 dans 1000 cm³ d'éthanol, on ajoute sous agitation à environ 20°C une solution de 3,78 g d'iodure de potassium dans 20 cm³ d'eau. La suspension est chauffée à 77°C et on ajoute à cette température, en 60 minutes, 30 cm³ d'eau oxygénée à 33% en poids. Le mélange réactionnel est maintenu à reflux 30 minutes supplémentaires puis est refroidi à environ 20°C. A cette température on verse, en 5 minutes, une solution de 11,4 g de thiosulfate de sodium dans 50 cm³ d'eau. Le précipité obtenu est essoré à 20°C environ et lavé par 2 fois 300 cm³ d'eau. Le produit humide obtenu est séché sous pression réduite (20 kPa) à environ 60°C. On isole 73 g d'éthoxy-3 difluoro-7,8 méthyl-1 oxo-4 dihydro-1,4 benzo[b]naphtyridine-1,8 sous forme d'un solide blanc fondant à une température supérieure à 270°C.

### La chloro-2 éthoxycarbonyl-3 difluoro-6,7 quinoléïne peut être préparée de la manière suivante :

A 200 cm³ de chlorure de phosphoryle, on ajoute en 10 minutes, sous agitation, à 20°C, 50 g d'éthoxycarbonyl-3 difluoro-6,7 carbostyrile. la suspension est chauffée à une température voisine de 70°C et maintenue à cette température pendant 3 heures. Après refroidissement à environ 10°C, la solution obtenue est versée, sous agitation, dans un mélange de 1 000 cm³ d'eau et 1 000 g de glace pilée. On laisse la température remonter à environ 20°C et extrait par 2 fois 500 cm³ de dichlorométhane. Les extraits organiques réunis sont lavés par 1 000 cm³ d'eau, 1 000 cm³ d'eau additionnés de bicarbonate de sodium jusqu'à pH=7, séchés sur sulfate de sodium, filtrés et concentrés à sec sous pression réduite (20kPa) à environ 40°C. On obtient 45,6 g de chloro-2 éthoxycarbonyl-3 difluoro-6,7 quinoléïne sous forme d'un solide beige fondant à 108°C qui est utilisé sans autre purification pour les étapes ultérieures.

### L'éthoxycarbonyl-3 difluoro-6,7 carbostyrile peut être obtenu de la manière suivante :

A une solution de 56,5 g de difluoro-3,4 nitro-6 benzaldéhyde dans 92 cm³ d'anhydride acétique, on ajoute, sous agitation, en dix minutes, 62,8 g de malonate d'éthyle et 51 g de bicarbonate de sodium. La suspension est maintenue 1 heure à environ 20°C puis chauffée 3 heures à une température d'environ 75°C. A cette température on verse, en 30 minutes, 400 cm³ d'acide acétique glacial puis 65 cm³ d'eau. On laisse la température se stabiliser à 50°C environ et l'on agite encore 30 minutes à cette température. On ajoute 39 g de fer en poudre, par fractions, en 2 heures, au mélange réactionnel. La température s'élève à environ 85°C et la suspension est maintenue 1 heure supplémentaire à cette température. Les sels de fer formés sont essorés a 80°C environ, puis lavés par 2 fois 150 cm³ d'acide acétique glacial. Le filtrat et les phases acides de lavage sont réunis et additionnés à 700 cm³ d'eau. le précipité obtenu est essoré à 20°C environ et lavé par 3 fois 500 cm³ d'eau. Le produit humide obtenu est séché sous pression réduite (20 kPa) à 50°C environ. On obtient 53,5 g d'éthoxycarbonyl-3 difluoro-6,7 carbostyrile sous forme d'un solide crème fondant à 242°C.

### Le difluoro-3,4 nitro-6 benzaldéhyde est préparé de la manière suivante :

A 520 cm³ d'acide sulfurique, sous agitation, refroidi, à 0°C, on ajoute, en 30 minutes, 60 cm³ d'acide nitrique fumant. A la solution obtenue, on ajoute, en 30 minutes, à environ 0°C, 100 g de difluoro-3,4 benzaldéhyde. On laisse remonter la température à environ 20°C et agite encore 3 heures à cette température. Après refroidissement à environ 5°C, le mélange réactionnel est versé, en 30 minutes, sous forte agitation, dans 1200 g de glace pilée. On laisse la température remonter à environ 20°C et extrait par 2 fois 600 cm³ de toluène. Les extraits organiques réunis sont lavés par 3 fois 1 000 cm³ d'eau, et concentrées sous pression réduite (20kPa) à 50°C. On obtient 113 g de difluoro-3,4 nitro-6 benzaldéhyde sous forme d'une huile brune qui est utilisée telle quelle dans la synthèse ultérieure.

### Exemple 2

### Préparation de l'éthoxycarbonyl-3 difluoro-6,7 (N-éthyl N-β éthoxycarbonyléthyl) amino-2 quinoléïne :

A une solution de 10 g de chloro-2 éthoxycarbonyl-3 difluoro-6,7 quinoléïne et 9,7 g de N-éthyl, N-β éthoxycarbonyléthyl amine dans 120 cm³ de toluène, on ajoute 7,8 g de carbonate de sodium. La suspension obtenue est chauffée à environ 90°C puis agitée 4 heures à cette température. Le mélange réactionnel est ensuite refroidi à environ 20°C puis lavé par 3 fois 100 cm³ d'eau. La phase organique est concentrée à sec sous pression réduite (20 kPa) à environ 50°C. On obtient 13 g d'éthoxycarbonyl-3 difluoro-6,7 (N-éthyl N-β éthoxycarbonyléthyl) amino-2 quinoléïne sous forme d'une huile qui est utilisée sans autre purification pour l' étape ultérieure.

### Préparation de l'éthoxycarbonyl-3 difluoro-7,8 éthyl-1 oxo-4 tétrahydro-1,2,3,4 benzo[b]naphtyridine-1,8 :

A une solution de 16,1 g d'éthylate de sodium porté à reflux dans 600 cm³ d'éthanol absolu on verse, en 60 minutes, une solution de 68 g d'éthoxycarbonyl-3 difluoro-6,7 (N-éthyl N-β éthoxycar-bonyléthyl) amino-2 quinoléïne dans 200 cm³ d'éthanol absolu. La suspension obtenue, toujours, à reflux, est agitée 60 minutes supplémentaires. On verse, ensuite, en 30 minutes, 20 cm³ d'acide acétique glacial. Le mélange réactionnel est agité 15 minutes supplémentaires puis on verse en 45 minutes, toujours à reflux, 400 cm³ d'eau. La suspension obtenue est refroidie à 20°C environ. Le précipité obtenu est essoré à 20°C environ et lavé par deux fois 200 cm³ d'eau. Le produit humide est séché sous pression réduite (20 kPa) à environ 50°C. On isole 52,4 g d'éthoxycarbonyl-3 difluoro-7,8 éthyl-1 oxo-4 tétrahydro-1,2,3,4 benzo[b]naphtyridine-1,8 sous forme d'un solide jaune d'or fondant à 152°C.

### Préparation de l'éthoxycarbonyl-3 difluoro-7,8 éthyl-1 oxo-4 dihydro-1,4 benzo[b]naphtyridine-1,8 :

A une suspension de 33 g d'éthoxycarbonyl-3 difluoro-7,8 éthyl-1 oxo-4 tétrahydro-1,2,3,4 benzo[b]naphtyridine-1,8 dans 1000 cm³ d'éthanol, on ajoute sous agitation à environ 20°C une solution de 1,7 g d'iodure de potassium dans 10 cm³ d'eau. La suspension est chauffée à 77°C et on verse à cette température, en 30 minutes, 12,7 cm³ d'eau oxygénée à 33 % en poids. Le mélange réactionnel est maintenu à reflux 30 minutes supplémentaires puis est refroidi à environ 20°C. A cette température on verse en 5 minutes une solution de 6 g de thiosulfate de sodium dans 20 cm³ d'eau. Le précipité obtenu est essoré à 20°C environ et lavé par deux fois 150 cm³ d'eau. Le produit humide obtenu est séché sous pression réduite (20 kPa) à environ 50°C. On isole 28,7 g d'éthoxycarbonyl-3 difluoro-7,8 éthyl-1 oxo-4 dihydro-1,4 benzo[b]naphtyridine-1,8 sous forme d'une solide jaune clair fondant à 270°C.

### Exemple 3

### Préparation de l'éthoxycarbonyl-3 difluoro-6,7 (N-cyclopropyl N-β éthoxycarbonyléthyl) amino-2 quinoléïne :

A une solution de 3,48 g de chloro-2 éthoxycarbonyl-3 difluoro-6,7 quinoléïne et 3 g de N-cyclopropyl N-β éthoxycarbonyléthyl amine dans 10 cm³ de toluène, on ajoute 3 g de carbonate de sodium. La suspension obtenue est chauffée à reflux puis agitée 15 heures à cette temprérature. Le mélange réactionnel est ensuite refroidi à environ 20°C puis on ajoute 30 cm³ d'eau et 4,5 cm³ d'acide acétique. Après décantation, le mélange réactionnel est lavé par 2 fois 10 cm³ d'eau. La phase organique est concentrée à sec sous pression réduite (20 kPa) à environ 50°C. On obtient 3,3 g d'éthoxycarbonyl-3 difluoro-6,7 (N-cyclopropyl N-β éthoxycarbonyléthyl) amino-2 quinoléïne brute sous forme d'une huile qui est utilisée sans autre purification pour l'étape ultérieure.

### Préparation de l'éthoxycarbonyl-3 difluoro-7,8 cyclopropyl-1 oxo-4 tétrahydro-1,2,3,4 benzo[b]naphtyridine-1,8 :

A une solution de 1,6 g d'éthylate de sodium porté à reflux dans 40 cm³ d'éthanol absolu on ajoute, en 60 minutes, une solution d' éthoxycarbonyl-3 difluoro-6,7 (N-cyclopropyl N-β éthoxycarbonyléthyl) amino-2 quinoléïne dans 20 cm³ d'éthanol absolu. La solution obtenue est agitée 60 minutes supplémentaires, à reflux. On verse ensuite, en 10 minutes, 2,6 cm³ d'acide acétique glacial. Le mélange réactionnel est agité 15 minutes supplémentaires puis on verse en 5 minutes, toujours à reflux, 26 cm³ d'eau. La suspension obtenue est refroidie à 20°C environ. Le précipité est essoré à 20°C environ et lavé par deux fois 10 cm³ d'eau. Le produit humide est séché sous pression réduite (20 kPa) à environ 60°C. on isole 1,25 g d'éthoxycarbonyl-3 difluoro-7,8 cyclopropyl-1 oxo-4 tétrahydro-1,2,3,4 benzo[b]naphtyridine-1,8 brute sous forme d'un solide jaune fondant à 172°C.

### Préparation de l'éthoxycarbonyl-3 difluoro-7,8 cyclopropyl-1 oxo-4 dihydro-1,4 benzo[b]naphtyridine-1,8 :

A une suspension de 1 g d'éthoxycarbonyl-3 difluoro-7,8 cyclopropyl-1 oxo-4 tétrahydro-1,2,3,4 benzo[b]naphtyridine-1,8 dans 14 cm³ d'éthanol, on ajoute sous agitation à environ 20°C une solution de 0,053 g d'iodure de potassium dans 0,5 cm³ d'eau. La suspension est chauffée à 77°C et on verse à cette température, en 5 minutes, 0,5 cm³ d'eau oxygénée à 33 % en poids. Le mélange réactionnel est maintenu à reflux 60 minutes supplémentaires puis refroidi à environ 20°C. A cette température on verse en 5 minutes, 1,06 cm³ d'une solution 1N de thiosulfate de sodium. Le précipité obtenu est essoré à 20°C environ et lavé par 2 fois 10 cm³ d'eau. Le produit humide obtenu est séché sous pression réduite (20 kPa) à environ 60°C. On isole 0,7 g d'éthoxycarbonyl-3 difluoro-7,8 cyclopropyl-1 oxo-4 dihydro-1,4 benzo[b]naphtyridine-1,8 brute sous forme d'un solide blanc ocre fondant à 210°C.

### Exemple 4

### L'éthoxycarbonyl-3 difluoro-6,7 (N-méthyl N-β-cyanoéthyl amino)-2 quinoléine est préparée de la manière suivante :

A une solution de 16,3 g de chloro-2 éthoxycarbonyl-3 difluoro-6,7 quinoléine et 10 g de N-méthyl N-β-cyanoéthyl amine dans 160 cm³ de toluène, on ajoute 19,08 g de carbonate de sodium. La suspension obtenue est chauffée à reflux puis agitée 4 heures à cette température. Le mélange réactionnel est ensuite refroidi à environ 20°C puis lavé par 3 fois 50 cm3 d'eau. La phase organique est concentrée à sec sous pression réduite (20 kPa) à environ 50°C. On obtient 19,17 g d'éthoxycarbonyl-3 difluoro-6,7 (N-méthyl N-β-cyanoéthyl amino) -2 quinoléine sous forme d'une huile qui est utilisée sans autre purification pour les étapes ultérieures.

### La cyano-3 difluoro-7,8 méthyl-1 oxo-4 tétrahydro-1,2,3,4 benzo[b]naphtyridine-1,8 est préparée de la manière suivante :

A une solution de 8,74 g de terbutylate de potassium dans 200 cm³ de tétrahydrofuranne refroidi à -10°C on verse, en 60 minutes, une solution de 19,17 g d'éthoxycarbonyl-3 difluoro-6,7 (N-méthyl N-β-cyanoéthyl amino)-2 quinoléine dans 50 cm³ de tétrahydrofuranne. La suspension obtenue est agitée toujours à -10°C durant 30 minutes supplémentaires. On verse ensuite 4 cm3 d'acide acétique glacial. Le tétrahydrofuranne est évaporé sous pression réduite (20 kPa). Le mélange brut réactionnel est repris par 200 cm³ d'un mélange hydroalcoolique éthanol/eau (70/30 vol/vol). Le précipité obtenu est filtré, lavé 2 fois par 50 cm3 d'eau, puis séché sous pression réduite (20 kPa). On isole 16,1 g de cyano-3 difluoro-7,8 méthyl-1 oxo-4 tétrahydro-1,2,3,4 benzo[b]naphtyridine-1,8 sous forme d'un solide jaune d'or fondant à 144°C.

### La cyano-3 difluoro-7,8 méthyl-1 oxo-4 dihydro-1,4 benzo[b]naphtyridine-1,8 est préparée de la manière suivante :

A une suspension de 8,6 g de cyano-3 difluoro-7,8 méthyl-1 oxo-4 tétrahydro-1,2,3,4 benzo[b]naphtyridine-1,8 dans 350 cm³ d'éthanol, on ajoute sous agitation à environ 20°C une solution de 0,47 g d'iodure de potassium dans 5 cm³ d'eau. La suspension est chauffée à 77°C et additionnée à cette température, en 10 minutes, de 4 cm³ d'eau oxygénée à 33 % en poids. Le mélange réactionnel est maintenu à reflux 30 minutes supplémentaires puis est refroidi à environ 20°C. A cette température on ajoute, en 5 minutes, 10 cm³ d'une solution de thiosulfate de sodium 1N. Le précipité obtenu est essoré à 20°C environ et lavé par deux fois 20 cm3 d'eau. Le produit humide obtenu est séché sous pression réduite (20 kPa) à environ 50°C. On isole 8 g de cyano-3 difluoro-7,8 méthyl-1 oxo-4 dihydro-1,4 benzo[b]naphtyridine-1,8 sous forme d'un solide jaune clair fondant à 380°C.

### Exemple 5

### L'éthoxycarbonyl-3 difluoro-6,7 [N-méthyl N-β (N',N' -diméthylaminocarbonyléthyl) amino]-2 quinoléine est préparée de la manière suivante :

A une solution de 26 g de chloro-2 éthoxycarbonyl-3 difluoro-6,7 quinoléine et 25 g de N-méthyl N-β (N',N'-diméthylaminocarbonyléthyl) amine dans 300 cm³ de toluène, on ajoute 31 g de carbonate de sodium. La suspension obtenue est chauffée à reflux puis agitée 2 heures 30 minutes à cette température. Le mélange réactionnel est ensuite refroidi à environ 20°C puis lavé par 3 fois 100 cm³ d'eau. La phase organique est concentrée à sec sous pression réduite (20 kPa) à environ 50°C. On obtient 35 g d'éthoxycarbonyl-3 difluoro-6,7 [N-méthyl N-β (N',N'-diméthylaminocarbonyl éthyl) amino]-2 quinoléine sous forme d'une huile qui est utilisée sans autre purification pour les étapes ultérieures.

### La N,N-diméthyl difluoro-7,8 méthyl-1 oxo-4 tétrahydro-1,2,3,4 benzo[b]naphtyridine-1,8 carboxamide-3 est préparée de la manière suivante :

A une solution de 15,7 g de terbutylate de potassium dans 150 cm³ de tétrahydrofuranne refroidi à 0°C on ajoute, en 75 minutes, une solution de 35 g d'éthoxycarbonyl-3 difluoro-6,7 N-méthyl N-β (N',N'-diméthylaminocarbonyl éthyl) amino-2 quinoléine dans 150 cm³ de tétrahydrofuranne. La suspension obtenue est ensuite agitée à 0°C durant 30 minutes supplémentaires puis on ajoute 8 cm³ d'acide acétique glacial. Le tétrahydrofuranne est évaporé sous pression réduite (20 kPa). Le mélange brut réactionnel est repris par 200 cm³ d'un mélange hydroalcoolique éthanol/eau (70/30 vol/vol). Le précipité obtenu est filtré, lavé 3 fois par 100 cm³ d'eau, puis séché sous vide (20 kPa).

On isole 25 g de N,N-diméthyl difluoro-7,8 méthyl-1 oxo-4 tétrahydro-1,2,3,4 benzo[b]naphtyridine-1,8 carboxamide-3 sous forme d'un solide jaune citron fondant à 206°C.

### La N,N-diméthyl difluoro-7,8 méthyl-1 oxo-4 dihydro-1,4 benzo[b]naphtyridine-1,8 carboxamide-3 est préparée de la manière suivante :

A une suspension de 25 g de N,N-diméthyl difluoro-7,8 méthyl-1 oxo-4 tétrahydro-1,2,3,4 benzo[b]naphtyridine-1,8 carboxamide-3 dans 1000 cm³ d'éthanol, on ajoute sous agitation à environ 20°C une solution de 1,35 g d'iodure de potassium dans 10 cm³ d'eau. La suspension est chauffée à 77°C et on verse à cette température, en 20 minutes, 25 cm³ d'eau oxygénée à 33 % en poids. Le mélange réactionnel est maintenu à reflux 1 heure 30 minutes supplémentaire puis est refroidi à environ 20°C. A cette température on coule,en 5 minutes, 30 cm³ d'une solution de thiosulfate de sodium 1N. Le précipité obtenu est essoré à 20°C environ et lavé par deux fois 60 cm³ d'eau. Le produit humide obtenu est séché sous pression réduite (20 kPa) à environ 50°C. On isole 19,5 g de N,N-diméthyl difluoro-7,8 méthyl-1 oxo-4 dihydro-1,4 benzo[b]naphtyridine-1,8 carboxamide-3 sous forme d'un solide jaune clair fondant à 324°C.

### Exemple 6

### L'éthoxycarbonyl-3 difluoro-6,7 (N-méthyl N-β aminocarbonyléthyl amino)-2 quinoléine est préparée de la manière suivante :

A une solution de 4 g de chloro-2 éthoxycarbonyl-3 difluoro-6,7 quinoléine et 3 g de N-méthyl N-β-aminocarbonyléthyl amine dans 40 cm³ de toluène, on ajoute 4,4 g de carbonate de sodium. La suspension obtenue est chauffée à reflux puis agitée 2 heures 30 minutes à cette température. Le mélange réactionnel est ensuite refroidi à environ 20°C puis lavé par 3 fois 25 cm³ d'eau. La phase organique est concentrée à sec sous pression réduite (20 kPa) à environ 50°C. On obtient 4,7 g d'éthoxycarbonyl-3 difluoro-6,7 (N-méthyl N-β-aminocarbonyléthyl amino)-2 quinoléine sous forme d'une huile qui est utilisée sans autre purification pour les étapes ultérieures.

### La carboxamide-3 difluoro-7,8 méthyl-1 oxo-4 tétrahydro-1,2,3,4 benzo[b]naphtyridine-1,8 est préparée de la manière suivante :

A une solution de 1,8 g de terbutylate de potassium dans 50 cm³ de tétrahydrofuranne refroidi à 0°C on ajoute, en 30 minutes, une solution de 4,23 g d'éthoxycarbonyl-3 difluoro-6,7 (N-méthyl N-β- aminocarbonyléthyl amino) -2 quinoléine dans 20 cm³ de tétrahydrofuranne. La suspension obtenue est ensuite agitée à °C durant 30 minutes supplémentaires puis on verse 2 cm³ d'acide acétique glacial. Le tétrahydrofuranne est évaporé sous pression réduite (20 kPa). Le brut réactionnel est repris par 10 cm³ d'un mélange hydroalcoolique éthanol/eau ( 70/30 vol/vol). Le précipité obtenu est filtré, lavé 3 fois par 10 cm³ d'eau, puis séché sous pression réduite (20 kPa). On isole 1,6 g de carboxamide-3 difluoro-7,8 méthyl-1 oxo-4 tétrahydro-1,2,3,4 benzo[b]naphtyridine-1,8 sous forme d'un solide jaune fondant à 182°C.

### La carboxamide-3 difluoro-7,8 méthyl-1 oxo-4 dihydro-1,4 benzo[b]naphtyridine-1,8 est préparée de la manière suivante :

A une suspension de 1,3 g de carboxamide-3 difluoro-7,8 méthyl-1 oxo-4 tétrahydro-1,2,3,4 benzo[b]naphtyridine-1,8 dans 25 cm³ d'éthanol, on ajoute sous agitation à environ 20°C une solution de 0,1 g d'iodure de potassium dans 1 cm³ d'eau. La suspension est chauffée à 77°C et additionnée à cette température, en 5 minutes, de 1,5 cm³ d'eau oxygénée à 33 % en poids. Le mélange réactionnel est maintenu à reflux 1 heure 30 minutes supplémentaire puis est refroidi à environ 20°C. A cette température on ajoute 1 cm³ d'une solution de thiosulfate de sodium 1N. Le précipité obtenu est essoré à 20°C environ et lavé par deux fois 5 cm³ d'eau. Le produit humide obtenu est séché sous pression réduite (20 kPa) à environ 50°C. On isole 1,1 g de carboxamide-3 difluoro-7,8 méthyl-1 oxo-4 dihydro-1,4 benzo[b]naphtyridine-1,8 sous forme d'un solide orangé fondant à 318°C.

### Exemple 7

### En opérant comme décrit précédemment à l'exemple 5, on prépare les produits suivants :

- N,N-diméthyl difluoro-7,8 éthyl-1 oxo-4 dihydro-1,4 benzo[b]naphtyridine-1,8 carboxamide-3 ;
- N, N-diméthyl difluoro-7,8 cyclopropyl-1 oxo-4 dihydro-1,4 benzo[b]naphtyridine-1,8 carboxamide-3 ;
- N,N-diéthyl difluoro-7,8 méthyl-1 oxo-4 dihydro-1,4 benzo[b]naphtyridine-1,8 carboxamide-3 ;
- N,N-diéthyl difluoro-7,8 éthyl-1 oxo-4 dihydro-1,4 benzo[b]naphtyridine-1,8 carboxamide-3 ;
- N,N-diéthyl difluoro-7,8 cyclopropyl-1 oxo-4 dihydro-1,4 benzo[b]naphtyridine-1,8 carboxamide-3 ;
- (difluoro-7,8 méthyl-1 oxo-4 dihydro-1,4 benzo[b]naphtyridine-1,8 carbonyl-3)-1 pyrrolidine ;
- (difluoro-7,8 éthyl-1 oxo-4 dihydro-1,4 benzo[b]naphtyridine-1,8 carbonyl-3)-1 pyrrolidine ;
- (difluoro-7,8 cyclopropyl-1 oxo-4 dihydro-1,4 benzo[b]naphtyridine-1,8 carbonyl-3)-1 pyrrolidine ;
- (difluoro-7,8 méthyl-1 oxo-4 dihydro-1,4 benzo[b]naphtyridine-1,8 carbonyl-3)-1 pipéridine ;
- (difluoro-7,8 éthyl-1 oxo-4 dihydro-1,4 benzo[b]naphtyridine-1,8 carbonyl-3)-1 pipéridine ;
- (difluoro-7,8 cyclopropyl-1 oxo-4 dihydro-1,4 benzo[b]naphtyridine-1,8 carbonyl-3)-1 pipéridine ;
- (difluoro-7,8 méthyl-1 oxo-4 dihydro-1,4 benzo[b]naphtyridine-1,8 carbonyl-3)-1 morpholine ;
- (difluoro-7,8 éthyl-1 oxo-4 dihydro-1,4 benzo[b]naphtyridine-1,8 carbonyl-3)-1 morpholine ;
- (difluoro-7,8 cyclopropyl-1 oxo-4 dihydro-1,4 benzo[b]naphtyridine-1,8 carbonyl-3) -1 morpholine ;
- (difluoro-7,8 méthyl-1 oxo-4 dihydro-1,4 benzo[b]naphtyridine-1,8 carbonyl-3)-1 thiomorpholine ;
- (difluoro-7,8 éthyl-1 oxo-4 dihydro-1,4 benzo[b]naphtyridine-1,8 carbonyl-3)-1 thiomorpholine ;
- (difluoro-7,8 cyclopropyl-1 oxo-4 dihydro-1,4 benzo[b]naphtyridine-1,8 carbonyl-3)-1 thiomorpholine ;
- (difluoro-7,8 méthyl-1 oxo-4 dihydro-1,4 benzo[b]naphtyridine-1,8 carbonyl-3)-1 méthyl-4 pipérazine ;
- (difluoro-7,8 éthyl-1 oxo-4 dihydro-1,4 benzo[b]naphtyridine-1,8 carbonyl-3)-1 méthyl-4 pipérazine ;
- (difluoro-7,8 cyclopropyl-1 oxo-4 dihydro-1,4 benzo[b]naphtyridine-1,8 carbonyl-3) -1 méthyl-4 pipérazine.

Les produits de formule générale (I) obtenus aux exemples précédents peuvent être utilisés comme décrit ci-après :

### Exemple d'utilisation 1

### La cyano-3 fluoro-7 méthyl-1 (méthyl-4 pipérazinyl-1)-8 oxo-4 dihydro-1,4 benzo[b]naphtyridine-1,8 est préparée de la manière suivante :

Une suspension de 2,1 g de cyano-3 difluoro-7,8 méthyl-1 oxo-4 dihydro-1,4 benzo[b]naphtyridine-1,8 dans 100 cm³ de diméthylsulfoxyde est chauffée à 80°C en présence de 2 cm³ de N-méthyl pipérazine. Le mélange réactionnel est maintenu à cette température durant 8 heures. La solution obtenue est refroidie à température ambiante et agitée à cette température durant 15 heures. Le précipité formé est filtré, lavé par 3 fois 20 cm³ d'eau, et séché sous vide (20 kPa) à 50°C. On obtient 2,6 g de cyano-3 fluoro-7 méthyl-1 (méthyl-4 pipérazinyl-1)-8 oxo-4 benzo[b]naphtyridine-1,8 sous forme d'un précipité jaune fondant à 335°C.

### L'acide fluoro-7 méthyl-1 (méthyl-4 pipérazinyl-1)-8 oxo-4 dihydro-1,4 benzo[b]naphtyridine-1,8 carboxylique-3 est préparé de la manière suivante :

Une suspension de 2 g de cyano-3 fluoro-7 méthyl-1 (méthyl-4 pipérazinyl-1)-8 oxo-4 dihydro-1,4 benzo[b]naphtyridine-1,8 est chauffé à reflux dans 40 cm³ d'acide chlorhydrique 12N. Le mélange réactionnel est maintenu à cette température durant 15 heures. La solution obtenue est refroidie à température ambiante. Le produit qui cristallise est filtré, lavé à l'eau jusqu'à neutralité, et séché sous pression réduite (20 kPa) à 50°C. On obtient 1,5 g d'acide fluoro-7 méthyl-1 (méthyl-4 pipérazinyl-1)-8 oxo-4 dihydro-1,4 benzo[b]naphtyridine-1,8 carboxylique-3 monochlorhydrate sous forme de cristaux jaunes fondant à 290°C (décomposition).

### Exemple d'utilisation 2

Une suspension de 2,96 g de difluoro-7,8 N,N-diméthyl oxo-4 méthyl-1 dihydro-1,4 benzo[b]naphtyridine-1,8 carboxamide-3, de 1,12 g de méthyl-1 pipérazine et de 1,55 g de carbonate de potassium dans 100 cm³ de diméthylsulfoxyde est chauffée 5 heures, sous agitation, à environ 80°C. Après refroidissement à environ 20°C, le mélange réactionnel est additionné de 100 cm³ d'eau ; l'insoluble est essoré, lavé par 2 fois 30 cm³ d'eau et 2 fois 30 cm³ d'éthanol.

On obtient 2,3 g de N,N diméthyl fluoro-7 méthyl-1 (méthyl-4 pipérazinyl-1)-8 oxo-4 dihydro-1,4 benzo[b]naphtyridine-1,8 carboxamide-3 sous forme d'un solide jaune se décomposant à 275°C.

Une solution de 0,5 g de N,N-diméthyl fluoro-7 méthyl-1 (méthyl-4 pipérazinyl-1)-8 oxo-4 dihydro-1,4 benzo[b]naphtyridine-1,8 carboxamide-3 dans 10 cm³ d'acide chlorhydrique aqueux 6N est chauffée, sous agitation à environ 95°C, pendant 5 heures. Après refroidissement à environ 20°C, l'insoluble est essoré, lavé par 3 fois 20 cm³ d'eau et 2 fois 10 cm³ d'éthanol.

Le produit obtenu est mis en suspension dans 30 cm³ d'eau ; on ajoute 0,6 cm³ de potasse aqueuse N et agite 1 heure à environ 20°C. L'insoluble est essoré, lavé par 2 fois 20 cm³ d'eau et 2 fois 10 cm³ d'éthanol. Après recristallisation dans 15 cm³ diméthyformamide, on obtient 0,15 g d'acide fluoro-7 méthyl-1 (méthyl-4 pipérazinyl-1)-8 oxo-4 dihydro-1,4 benzo[b]naphtyridine-1,8 carboxylique-3 sous forme d'un solide jaune se décomposant à 354°C.

### Exemple d'utilisation 3

Une suspension de 1,3 g de difluoro-7,8 méthyl-1 oxo-4 dihydro-1,4 benzo[b]naphtyridine-1,8 carboxamide-3, de 0,54 g de méthyl-1 pipérazine et de 0,75 g de carbonate de potassium dans 25 cm³ de diméthylsulfoxyde est chauffée à environ 80°C pendant 6 heures. Après refroidissement à une température voisine de 20°C, le mélange réactionnel est additionné de 100 cm³ d'eau. L'insoluble est essoré, lavé par 2 fois 20 cm³ d'eau et 2 fois 20 cm³ d'éthanol.

Le produit obtenu est chromatographié sur 20 g de gel de silice (0,063-0,200 mm) en suspension dans un mélange de dichlorométhane à 10 % de méthanol. On élimine de impuretés réactionnelles par élution avec 500 cm³ de ce mélange de solvants. Le produit attendu est ensuite élué par 500 cm³ du même mélange de solvants. Après concentration à sec, sous pression réduite (20 kPa) à envion 40°C, le résidu solide est recristallisé dans 25 cm³ de diméthylformamide, essoré et lavé par 2 fois 30 cm³ d'éthanol à environ 70°C.

On obtient 0,6 g de fluoro-7 méthyl-1 (méthyl-4 pipérazine-1)-8 oxo-4 dihydro-1,4 benzo[b]naphtyridine-1,8 carboxamide-3 sous forme d'un solide jaune se décomposant à 265°C.

L'acide fluoro-7 (méthyl-4 pipérazinyl-1)-8 méthyl-1 oxo-4 dihydro-1,4 benzo[b]naphtyridine-1,8 carboxylique-3 est préparé dans les conditions de l'exemple d'utilisation 2, mais à partir de 0,3 g de fluoro-7 méthyl-1 (méthyl-4 pipérazinyl-1)-8 oxo-4 dihydro-1,4 benzo[b]naphtyridine-1,8 carboxamide-3. Après refroidissement à environ 20°C, le mélange réactionnel est additionné de 50 cm³ d'eau ; l'insoluble est essoré et lavé par 2 fois 10 cm³ d'eau.

Le produit obtenu est mis en suspension dans 20 cm³ d'eau, additionné de 0,4 cm³ d'une solution de potasse aqueuse N et agité pendant 1 heure à environ 20°C. L'insoluble est essoré, lavé par 3 fois 10 cm³ d'eau, 2 fois 10 cm³ d'éthanol et recristallisé dans 20 cm³ de diméthylformamide.

On obtient 0,17 g d'acide fluoro-7 méthyl-1 (méthyl-4 pipérazinyl-1)-8 oxo-4 dihydro-1,4 benzo[b]naphtyridine-1,8 carboxylique-3, sous forme d'un solide jaune se décomposant à 354°C.

## Revendications

1. Un procédé de préparation de benzo[b]naphtyridines de for-mule générale : 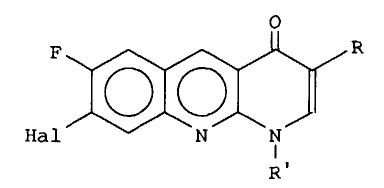 dans laquelle R est un radical carboxy, alcoyloxycarbonyle, cyano, carbamoyle, alcoylcarbamoyle, benzylcarbamoyle, hydroxyéthylcarbamoyle, dialcoylaminoéthylcarbamoyle ou dialcoylcarbamoyle dont les parties alcoyle peuvent éventuellement former avec l'atome d'azote auquel elles sont rattachées, un hétérocycle à 5 ou 6 chaînons contenant éventuellement un autre hétéroatome choisi parmi l'oxygène, le soufre ou l'azote et éventuellement substitué sur l'azote par un radical alcoyle, R' est un atome d'hydrogène ou un radical alcoyle, fluoroalcoyle, carboxyalcoyle, cycloalcoyle (contenant 3 à 6 atomes de carbone), fluorophényle, difluorophényle, alcoyloxy ou alcoylamino, et Hal est un atome d'halogène, ainsi que leurs sels lorsqu'ils existent, caractérisé en ce que l'on effectue les opérations suivantes :
1) condensation en milieu basique d'une amine de formule générale :
R'-NH-CH₂-CH₂-R''
dans laquelle R' est défini comme précédemment et R'' est un radical alcoyloxycarbonyle, cyano, carbamoyle, alcoylcarbamoyle, benzylcarbamoyle, hydroxyéthylcarbamoyle, dialcoylaminoéthylcarbamoyle ou dialcoylcarbamoyle dont les parties alcoyle peuvent éventuellement former avec l'atome d'azote auquel elles sont rattachées, un hétérocycle à 5 ou 6 chaînons contenant éventuellement un autre hétéroatome choisi parmi l'oxygène, le soufre ou l'azote et éventuellement substitué sur l'azote par un radical alcoyle, sur une chloro fluoro quinoléine de formule générale : 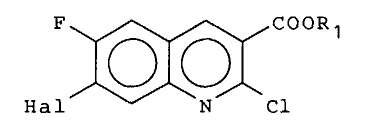 dans laquelle Hal est défini comme ci-dessus et R₁ est un atome d'hydrogène ou un radical alcoyle, étant entendu que les radicaux alcoyle cités ci-dessus sont droits ou ramifiés et contiennent 1 à 4 atomes de carbone,
2) cyclisation en milieu basique de la fluoroquinoléine, ainsi obtenue, de formule générale : 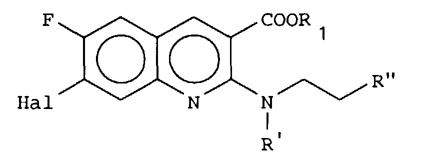 dans laquelle R', R'' et Hal sont définis comme précédemment et R₁ est défini comme ci-dessus en 1),
3) oxydation par l'eau oxygénée, éventuellement en présence d'iodure de potassium, de la tétrahydro-1,2,3,4 benzo[b]naphtyridine-1,8 de formule générale : 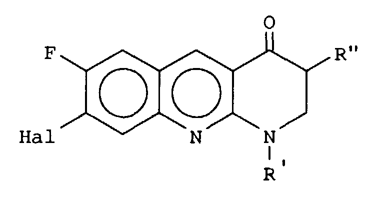 dans laquelle Hal, R' et R'' sont définis comme précédemment, puis éventuellement transformation de l'ester obtenu pour lequel R est alcoyloxycarbonyle, en un acide pour lequel R est un radical carboxy et éventuellement transformation de l'acide obtenu en un sel.

2. Un dérivé de benzonaphtyridine de formule générale : 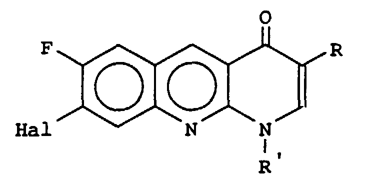 dans laquelle R' et Hal sont définis comme dans la revendication 1 et R est un radical cyano, carbamoyle, alcoylcarbamoyle, benzylcarbamoyle, hydroxyéthylcarbamoyle, dialcoylaminoéthylcarbamoyle ou dialcoylcarbamoyle dont les parties alcoyle peuvent éventuellement former avec l'atome d'azote auquel elles sont rattachées, un hétérocycle à 5 ou 6 chaînons contenant éventuellement un autre hétéroatome choisi parmi l'oxygène, le soufre ou l'azote et éventuellement substitué sur l'azote par un radical alcoyle.

## Claims

1. Process for preparing benzo[b]naphthyridines of general formula: 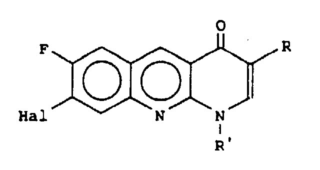 in which R is a carboxyl, alkyloxycarbonyl, cyano, carbamoyl, alkylcarbamoyl, benzylcarbamoyl or hydroxyethylcarbamoyl radical or a dialkylaminoethylcarbamoyl or dialkylcarbamoyl radical in which the alkyl portions, with the nitrogen atom to which they are attached, can optionally form a 5- or 6-membered heterocycle optionally containing another hetero atom chosen from oxygen, sulphur and nitrogen and optionally substituted on the nitrogen with an alkyl radical, R' is a hydrogen atom or an alkyl, fluoroalkyl, carboxylalkyl, cycloalkyl (containing 3 to 6 carbon atoms), fluorophenyl, difluorophenyl, alkyloxy or alkylamino radical and Hal is a halogen atom, as well as their salts where they exist, characterized in that the following operations are performed:
1) condensation in basic medium of an amine of general formula:
R'-NH-CH₂-CH₂-R''
in which R' is defined as before and R'' is an alkyloxycarbonyl, cyano, carbamoyl, alkylcarbamoyl, benzylcarbamoyl or hydroxyethylcarbamoyl radical or a dialkylaminoethylcarbamoyl or dialkylcarbamoyl radical in which the alkyl portions, with the nitrogen atom to which they are attached, can optionally form a 5- or 6-membered heterocycle optionally containing another hetero atom chosen from oxygen, sulphur and nitrogen and optionally substituted on the nitrogen with an alkyl radical, with a chlorofluoroquinoline of general formula: 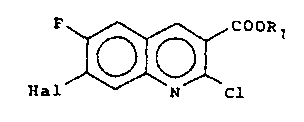 in which Hal is defined as above and R₁ is a hydrogen atom or an alkyl radical, on the understanding that the alkyl radicals mentioned above are unbranched or branched and contain 1 to 4 carbon atoms,
2) cyclization in basic medium of the fluoroquinoline thereby obtained, of general formula : 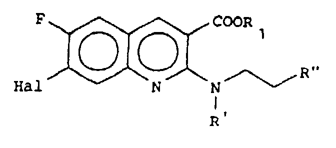 in which R', R'' and Hal are defined as before and R₁ is defined as above in 1),
3) oxidation with hydrogen peroxide, optionally in the presence of potassium iodide, of the 1,2,3,4-tetrahydrobenzo[b] [1,8] naphthyridine of general formula : 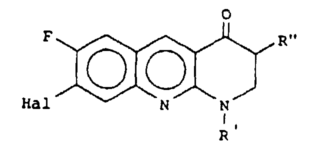 in which Hal, R' and R'' are defined as before, and then, optionally, conversion of the ester obtained for which R is alkyloxycarbonyl to an acid for which R is a carboxyl radical and, optionally, conversion of the acid obtained to a salt.

2. Benzonaphthyridine derivative of general formula : 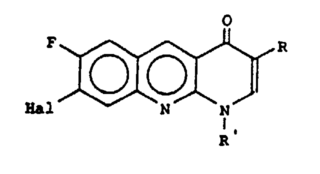 in which R' and Hal are defined as in claim 1 and R is a cyano, carbamoyl, alkylcarbamoyl, benzylcarbamoyl or hydroxyethylcarbamoyl radical or a dialkylaminoethylcarbamoyl or dialkylcarbamoyl radical in which the alkyl portions, with the nitrogen atom to which they are attached, can optionally form a 5- or 6-membered heterocycle optionally containing another hetero atom chosen from oxygen, sulphur and nitrogen and optionally substituted on the nitrogen with an alkyl radical.

## Patentansprüche

1. Ein Verfahren zur Herstellung von Benzo[b]naphthyridinen der allgemeinen Formel: 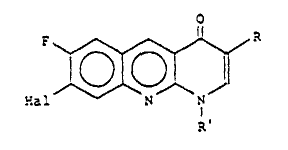 in der R ein Carboxy-, Alkyloxycarbonyl-, Cyano-, Carbamoyl-, Alkylcarbamoyl-, Benzylcarbamoyl-, Hydroxyethylcarbamoyl-, Dialkylaminoethylcarbamoyl- oder Dialkylcarbamoylrest ist, dessen Alkylteile gegebenenfalls mit dem Stickstoffatom, an das sie gebunden sind, einen Heterocyclus mit 5 oder 6 Kettengliedern, der gegebenenfalls ein weiteres unter Sauerstoff, Schwefel oder Stickstoff ausgewähltes Heteroatom enthält und gegebenenfalls am Stickstoff mit einem Alkylrest substituiert ist, bilden können, R' ein Wasserstoffatom oder ein Alkyl-, Fluoralkyl-, Carboxyalkyl-, Cycloalkyl- (mit 3 bis 6 Kohlenstoffatomen), Fluorphenyl-, Difluorphenyl-, Alkyloxy- oder Alkylaminorest ist und Hal ein Halogenatom ist, sowie deren Salzen, wenn sie existieren, dadurch gekennzeichnet, daß man die folgenden Vorgänge ausführt:
1) Kondensation in basischem Medium eines Amins der allgemeinen Formel:
R'-NH-CH₂-CH₂-R''
in der R' wie zuvor definiert ist und R'' ein Alkyloxycarbonyl-, Cyano-, Carbamoyl-, Alkylcarbamoyl-, Benzylcarbamoyl-, Hydroxyethylcarbamoyl-, Dialkylaminoethylcarbamoyl- oder Dialkylcarbamoylrest ist, dessen Alkylteile gegebenenfalls mit dem Stickstoffatom, an das sie gebunden sind, einen Heterocyclus mit 5 oder 6 Kettengliedern, der gegebenenfalls ein weiteres unter Sauerstoff, Schwefel oder Stickstoff ausgewähltes Heteroatom enthält und gegebenenfalls am Stickstoff mit einem Alkylrest substituiert ist, bilden können, an ein Chlorfluorchinolin der allgemeinen Formel: 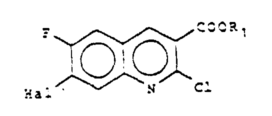 in der Hal wie oben definiert ist und R₁ ein Wasserstoffatom oder ein Alkylrest ist, wobei es sich versteht, daß die oben angeführten Alkylreste gerade oder verzweigt sind und 1 bis 4 Kohlenstoffatome enthalten,
2) Cyclisierung in basischem Medium des so erhaltenen Fluorchinolins der allgemeinen Formel: 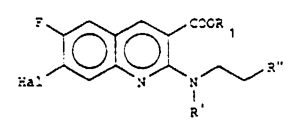 in der R', R'' und Hal wie zuvor definiert sind und R₁ wie oben in 1) definiert ist,
3) Oxidation durch Wasserstoffperoxid, gegebenenfalls in Gegenwart von Kaliumiodid, des Tetrahydro-1,2,3,4-benzo[b]naphthyridins-1,8 der allgemeinen Formel: 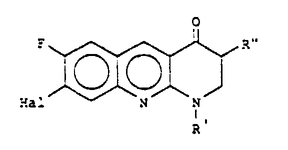 in der Hal, R' und R'' wie zuvor definiert sind, dann gegebenenfalls Umwandlung des erhaltenen Esters, für den R Alkyloxycarbonyl ist, in eine Säure, für die R ein Carboxyrest ist, und gegebenenfalls Umwandlung der erhaltenen Säure in ein Salz.

2. Ein Benzonaphthyridinderivat der allgemeinen Formel: 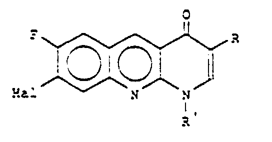 in der R' und Hal wie in Anspruch 1 definiert sind und R ein Cyano-, Carbamoyl-, Alkylcarbamoyl-, Benzylcarbamoyl-, Hydroxyethylcarbamoyl-, Dialkylamincethylcarbamoyl- oder Dialkylcarbamoylrest ist, dessen Alkylteile gegebenenfalls mit dem Stickstoffatom, an das sie gebunden sind, einen Heterocyclus mit 5 oder 6 Kettengliedern, der gegebenenfalls ein weiteres unter Sauerstoff, Schwefel oder Stickstoff ausgewähltes Heteroatom enthält und gegebenenfalls am Stickstoff mit einem Alkylrest substituiert ist bilden können.
